# EUROPEAN PATENT APPLICATION

(11) **EP 4 613 278 A1**
(43) Date of publication of application: **10.09.2025**
(21) Application number: 23886338.5
(22) Date of filing: 02.11.2023
(51) Int. Cl.: A61K 35/747, A61P 25/28, A23L 33/135

(54) **NOVEL USE OF LACTOBACILLUS DELBRUECKII SUBSP. LACTIS STRAIN**

(30) Priority: 02.11.2022 KR 20220144826; 07.06.2023 KR 20230073225
(71) Applicant: CKD Bio Corporation, Seoul 03742 (KR); Genome and Company, Seongnam-si, Gyeonggi-do 13486 (KR)
(72) Inventor: NOH, Hye-Ji, Ansan-si Gyeonggi-do 15373 (KR); CHA, Lina, Anyang-si Gyeonggi-do 14043 (KR); LEE, In Ock, Siheung-si Gyeonggi-do 14994 (KR); MOOK, Inhee, Seoul 04386 (KR); CHOI, Hyunjung, Seoul 02725 (KR); KIM, Kyung Hwan, Seoul 01703 (KR); KIM, Byoung-Kook, Seoul 08048 (KR); SHIN, Chang-Hun, Hwaseong-si Gyeonggi-do 18237 (KR); KIM, Hyerim, Seongnam-si Gyeonggi-do 13486 (KR); KIM, Hyeyoung, Seongnam-si Gyeonggi-do 13486 (KR); LEE, Suro, Seongnam-si Gyeonggi-do 13486 (KR); LEE, Hyerim, Seongnam-si Gyeonggi-do 13486 (KR); HONG, Riwon, Seongnam-si Gyeonggi-do 13486 (KR); HEO, Jinah, Seongnam-si Gyeonggi-do 13486 (KR)
(74) Representative: Rückerl, Florian
(86) International application number: PCT/KR2023/017436
(87) International publication number: WO 2024/096644

(57) **Abstract**

The present invention relates to a composition for preventing, alleviating or treating cognitive impairment or Alzheimer's disease, comprising *a Lactobacillus delbrueckii* subsp. *lactis* strain as an active ingredient. The present invention provides a composition for preventing, alleviating or treating cognitive impairment or Alzheimer's disease (AD), comprising a Lactobacillus delbrueckii subsp. lactis strain as an active ingredient. The strain of the present invention has the excellent effects of reducing amyloid beta protein (Aβ) or tau protein (Tau) and improving cognitive function, and thus can be effectively used as a composition for preventing, alleviating or treating cognitive impairment or AD.

## Description

### [Technical Field]

The present application claims the benefit of priority based on Korean Patent Application No. 10-2022-0144826, filed on November 2, 2022, and all contents disclosed in the document of the corresponding Korean patent application are incorporated herein as part of this specification.

The present invention relates to a composition for the prevention, amelioration, or treatment of cognitive impairment or Alzheimer's disease, comprising a *Lactobacillus delbrueckii* subsp. *lactis* strain as an active ingredient.

### [Background Art]

Alzheimer's disease (AD) is a degenerative brain disease in which cognitive function declines, and it belongs to the most common form of dementia. AD progressively deteriorates various cognitive functions such as memory, language ability, judgment, and thinking ability, causing serious problems and inconveniences in daily life, but the exact cause and mechanism of onset have not yet been elucidated. According to reports so far, the abnormal accumulation of amyloid beta protein (Aβ) and tau Protein (Tau) in brain tissue, the decrease of acetylcholine (ACh) due to the continuous action of acetylcholinesterase (AChE), genetic causes, and damage to nerve cells due to the deterioration of blood-brain barrier function and the resulting excessive activation of immune cells in the brain are recognized as the main causes of cognitive function decline. Therefore, most of the currently used AD therapeutics are drugs aimed at treating signal transmission problems between brain nerve cells, and research for therapeutic development has also been mainly limited to the brain.

It has recently been revealed through research that gut microorganisms are related to the onset or treatment of brain diseases. Specifically, gut microorganisms play an important role in bidirectional communication via the endocrine, nervous, and immune systems between the gut and the brain. That is, changes in the composition and metabolites of gut microorganisms directly affect not only appetite, sleep, and mood regulation but also brain functions such as memory and learning through the gut-brain axis mediated by systemic immunity, hormones, and neurotransmitters. As the correlation between the gut and the brain has become clear, research on the effects of gut microorganisms on brain diseases is being actively conducted.

However, in the case of lactic acid bacteria, which account for more than 80% of normal gut microorganisms, even microorganisms belonging to the same species exhibit strain specificity, showing different biological activities depending on the strain. Accordingly, the development of pharmaceuticals for treating cognitive impairment or AD using such microorganisms still remains challenging.

### [Prior Art Document]

### (Patent Document 1) Korean Patent No. 10-2128098 (published on June 30, 2020)

### [Technical Problem]

As a result of conducting various studies to prevent, improve, or treat cognitive impairment or Alzheimer's disease (AD) using gut microorganisms, the inventors have experimentally demonstrated that a *Lactobacillus delbrueckii* subsp. *lactis* strain improves intestinal barrier permeability, reduces amyloid beta protein (Aβ) and tau protein (Tau), which are markers of degenerative brain diseases such as AD, inhibits the activity of reactive oxygen species (ROS) or acetylcholinesterase (AChE) to protect nerve cells, reduces inflammatory responses, and improves short-term memory ability, spatial perception ability, or cognitive function in animal models, thereby completing the present invention.

### [Technical Solution]

The present invention provides a pharmaceutical composition for the prevention or treatment of cognitive impairment or Alzheimer's disease (AD), comprising a *Lactobacillus delbrueckii* subsp. *lactis* strain deposited under accession number KCTC14149BP as an active ingredient.

The term *"Lactobacillus delbrueckii* subsp. *lactis* strain" as used in the present specification refers to the strain deposited by the applicant with the Korea Research Institute of Bioscience and Biotechnology under accession number KCTC14149BP on March 3, 2020. The term *"Lactobacillus delbrueckii* subsp. *lactis* strain" in the present specification may be used interchangeably with *"Lactobacillus delbrueckii* subsp. *lactis", "Lactobacillus delbrueckii",* "microbial cells", or "strain".

The term "strain" as used in the present specification includes live cells, killed cells, and their cultures, lysates, extracts, and cytoplasmic fractions. In addition, it also includes materials subjected to post-treatment or post-processing such as filtration, concentration, drying, extraction, or freezing. The term "culture" as used in the present specification includes the strain itself obtained by culturing the strain in a medium for a certain period, metabolites of the strain, the entire medium, the culture broth from which the strain has been removed after cultivation, or the culture supernatant. The culturing method, extraction method, separation method, concentration method, drying method, and dilution method of the strain are not particularly limited.

The medium for culturing microbial cells generally includes milk proteins such as skim milk, whey, casein, sugars, yeast extract, and the like, and the culturing method may employ various general aerobic or anaerobic methods.

For example, the culture temperature may be set to 35-45°C. During cultivation, a neutralization culture method may be employed in which the pH of the medium is maintained at an acidic level, for example, pH 5 to 6, by using an alkali such as sodium hydroxide. In addition to such neutralization culture methods, any suitable culturing methods such as batch culture may be employed. After cultivation, the culture or its supernatant may be concentrated, dried, or diluted, as necessary.

In addition, the culture supernatant and the microbial cells may be separated using centrifugation or membrane separation methods, and the cell mass may be recovered in a concentrated state. Furthermore, ultrasonic treatment or enzymatic treatment may be applied to the microbial cells to extract components from the cells, and the culture broth, its supernatant, the microbial cells, or their extracts may be dried. These may be used as active ingredients of the composition according to the present invention.

The physical characteristics of the cell mass of the *Lactobacillus delbrueckii* subsp. *lactis* according to the present invention are as follows:
- Morphology of the bacterium: Gram-positive, rod-shaped or spherical
- Physiological properties: Optimal growth temperature is 36-38°C, optimal pH is 5.5-5.8, and it is facultative anaerobic. It is non-motile and does not form spores.
- Generally isolated from fermented milk or cheese, and considered safe for use in food (probiotics and dairy products).

The biochemical characteristics of the *Lactobacillus delbrueckii* subsp. *lactis* are shown in Table 1, and the 16S rRNA sequence of the strain is represented as SEQ ID NO: 1.

**[Table 1]**

| Source | | *L. lactis* |
|---|---|---|
| | | KCTC14149BP |
| Temoin | 0 | + |
| Glycerol | 1 | - |
| Erythritol | 2 | - |
| D-Arabinose | 3 | - |
| L-Arabinose | 4 | - |
| D-Ribose | 5 | - |
| D-Xylose | 6 | - |
| L-Xylose | 7 | - |
| D-Adonitol | 8 | - |
| Methyl-lolXylopyranoside | 9 | - |
| D-Galactose | 10 | - |
| D-Glucose | 11 | + |
| D-Fructose | 12 | + |
| D-Mannose | 13 | + |
| L-Sorbose | 14 | - |
| L-Rhamnose | 15 | - |
| Dulcitol | 16 | - |
| Inositol | 17 | - |
| D-Mannitol | 18 | - |
| D-Sorbitol | 19 | - |
| Methyl-tolMannopyranoside | 20 | - |
| Methyl-ranGlucopyranoside | 21 | - |
| N-Acetylglucosamine | 22 | + |
| Amygdaline | 23 | - |
| Arbutine | 24 | - |
| Esculine, citrate de fer | 25 | - |
| Salicine | 26 | - |
| D-Cellobiose | 27 | - |
| D-Maltose | 28 | w |
| D-Lactose | 29 | + |
| D-Melibiose | 30 | - |
| D-Saccharose | 31 | + |
| D-Trehalose | 32 | + |
| Inuline | 33 | - |
| D-Melezitose | 34 | - |
| D-Raffinose | 35 | - |
| Amidon | 36 | - |
| Glycogene | 37 | - |
| Xylitol | 38 | - |
| Gentiobiose | 39 | - |
| D-Turanose | 40 | - |
| D-Lyxose | 41 | - |
| D-Tagatose | 42 | - |
| D-Fucose | 43 | - |
| L-Fucose | 44 | - |
| D-Arabitol | 45 | - |
| L-Arabitol | 46 | - |
| Potassium Gluconate | 47 | - |
| Potassium 2-CetoGluconate | 48 | - |
| Potassium 5-CetoGluconate | 49 | - |

In the present invention, the *Lactobacillus delbrueckii* subsp. *lactis* may be used for the prevention or treatment of cognitive impairment or AD.

The term "cognitive impairment" as used in the present specification refers to diseases exhibiting symptoms of decline in memory, attention, spatial perception ability, language ability, judgment, or a combination thereof.

In one embodiment of the present invention, the cognitive impairment may be a disease caused by the accumulation of amyloid beta (Aβ) protein or tau (Tau) protein. The Aβ may be soluble or insoluble Aβ, and specific examples include Aβ₄₀, Aβ₄₂, or a combination thereof, but are not limited thereto.

The Tau may be soluble or insoluble Tau, may be phosphorylated Tau, and specific examples include phospho-Tau (Thr231), phospho-Tau (Ser202, Thr205), phospho-Tau (Thr181), phospho-Tau (Thr212, Ser214), phospho-Tau (Ser396), and phospho-Tau (Ser422), but are not limited thereto.

Diseases caused by the accumulation of Aβ may be one or more selected from the group consisting of AD, Parkinson's disease dementia, dementia with Lewy bodies, Huntington's dementia, preclinical Alzheimer's disease, and Down syndrome, and diseases caused by the accumulation of Tau may be one or more selected from the group consisting of corticobasal degeneration (CBD), progressive supranuclear palsy (PSP), Pick's disease, and frontotemporal dementia (FTD), but are not limited thereto.

The term "Alzheimer's disease (AD)" as used in the present specification refers to a degenerative brain disease that represents the most common form of dementia. AD involves progressive memory deterioration, and as the disease advances, a decline in various cognitive functions such as language, judgment, and reasoning ability ultimately leads to severe problems in daily life.

In the present invention, the *Lactobacillus delbrueckii* subsp. *lactis* inhibits the production of Aβ or Tau in brain tissue.

In one embodiment of the present invention, the composition inhibits the activity of reactive oxygen species (ROS) or acetylcholinesterase (AChE) and protects nerve cells.

In one embodiment of the present invention, the composition inhibits inflammatory responses by reducing inflammatory cytokines such as nitric oxide (NO) and tumor necrosis factor-α (TNF-α).

In one embodiment of the present invention, the composition improves short-term memory ability, spatial perception ability, or cognitive function.

In one embodiment of the present invention, the composition has the effect of reducing intestinal barrier permeability.

In one embodiment of the present invention, the composition shows an increase in one or more gut microorganisms selected from the group consisting of *Erysipelotrichaceae, Erysipelotrichales, Turicibacter, Peptostreptococcales Tissierellales, Anaerovoracaceae* and *Eubacterium xylanophilum.*

In one embodiment of the present invention, the composition shows a decrease in one or more gut microorganisms selected from the group consisting of *Peptoc Peptococcales* and *Peptococaceae.*

It is known that the gut microbiota potentially regulates neuroinflammation in various neurological conditions including multiple sclerosis, Parkinson's disease, and AD. Changes in the gut microbiota can alter microbially derived metabolites and peripheral immunity, which in relation to neurological diseases may potentially alter central nervous system immune responses (Sidhanth Chandra et al., Molecular Neurodegeneration 18 (9), February 1, 2023).

The pharmaceutical composition of the present invention may be administered to a subject in need of prevention or treatment of cognitive impairment or AD by comprising an effective amount of the above strain.

The term "administration" as used in the present specification means physically introducing the composition to a subject using any of the various methods and delivery systems known to those skilled in the art. The administration may be performed, for example, by oral administration, intravenous, intramuscular, subcutaneous, intraperitoneal, spinal, or other non-oral administration, for example, by injection or infusion, but is not limited thereto. The number of administrations may be, for example, a single time, multiple times, or over one or more extended periods.

The pharmaceutical composition of the present invention may be formulated in various oral or non-oral administration forms as described below, but is not limited thereto.

Oral administration forms include, for example, tablets, pills, hard/soft capsules, liquids, suspensions, emulsions, syrups, granules, and elixirs, and these formulations may use one or more diluents or excipients such as fillers, extenders, wetting agents, disintegrants, lubricants, binders, or surfactants, in addition to the above active ingredient. As disintegrants, agar, starch, alginic acid or its sodium salt, anhydrous monobasic calcium phosphate, and the like may be used; as lubricants, silica, talc, stearic acid or its magnesium or calcium salts, polyethylene glycol, and the like may be used; and as binders, magnesium aluminum silicate, starch paste, gelatin, tragacanth, methylcellulose, sodium carboxymethylcellulose, polyvinylpyrrolidone, and low-substituted hydroxypropylcellulose may be used. In addition, lactose, dextrose, sucrose, mannitol, sorbitol, cellulose, glycine, and the like may be used as diluents, and depending on the case, conventionally known additive mixtures, absorbents, colorants, flavoring agents, sweeteners, and the like may also be used together.

The composition may be sterilized or may contain preservatives, stabilizers, hydrating agents, or emulsifying promoters, salts for osmotic pressure regulation, buffers, and other therapeutically useful substances, and may be formulated according to conventional methods such as mixing, granulation, or coating methods.

The appropriate dosage of the pharmaceutical composition of the present invention may vary depending on factors such as formulation method, administration method, patient's age, weight, sex, pathological condition, food, time of administration, route of administration, excretion rate, and response sensitivity.

The pharmaceutical composition of the present invention may be prepared in a unit dosage form or contained in a multi-dose container by formulating it using pharmaceutically acceptable carriers and/or excipients according to methods easily carried out by those skilled in the art to which the invention pertains. In this case, the formulation may be in the form of a solution, suspension, syrup, or emulsion in an oil or aqueous medium, or in the form of an extract, powder, granule, tablet, or capsule, and may additionally include a dispersing agent or stabilizer. The pharmaceutical composition of the present invention may comprise only the strain or may further comprise appropriate carriers, excipients, or diluents conventionally used in the preparation of pharmaceutical compositions. Specifically, the carriers, excipients, and diluents that may be comprised in the pharmaceutical composition may be, for example, lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methylparaben, propylparaben, talc, magnesium stearate, and mineral oil, but are not limited thereto. These may be used alone or in combination of two or more. Additionally, the pharmaceutical composition may further comprise other commonly used additives such as antioxidants, wetting agents, sweeteners, flavoring agents, emulsifiers, suspending agents, preservatives, buffering agents, and/or bacteriostatic agents, if necessary. It may additionally comprise dispersants, surfactants, binders, lubricants, and the like, and may be formulated into powders, granules, tablets, liquids, capsules, suspensions, emulsions, syrups, creams, lotions, gels, ointments, aerosols, powder sprays, creams, suppositories, pills, and the like.

Another aspect of the present invention provides a method for the prevention or treatment of cognitive impairment or AD, comprising administering a *Lactobacillus delbrueckii* subsp. *lactis* strain deposited under accession number KCTC14149BP.

In the prevention or treatment method according to the present invention, each term has the same meaning as described in the pharmaceutical composition unless otherwise specified.

In the prevention or treatment method of cognitive impairment or AD according to the present invention, the *Lactobacillus delbrueckii* subsp. *lactis* strain deposited under accession number KCTC14149BP may be administered simultaneously, sequentially, or separately with other therapeutic agents to the subject.

The "simultaneous" administration refers to administering the *Lactobacillus delbrueckii* subsp. *lactis* strain of the present invention and other therapeutic agents as a single formulation at once, or administering the strain and other therapeutic agents as separate formulations at once, and in this case, the administration routes of the strain and the other therapeutic agents may differ. The "sequential" administration refers to administering the strain and other therapeutic agents relatively continuously, allowing only the minimal time required between administrations. The "separate" administration refers to administering the strain and other therapeutic agents with a certain time interval between administrations. The method of administration of the strain and the other therapeutic agents may be appropriately selected by a physician or expert in the art considering the therapeutic efficacy, side effects, and the like for the subject.

Another aspect of the present invention provides a food composition for the prevention or improvement of cognitive impairment or AD, comprising a *Lactobacillus delbrueckii* subsp. *lactis* strain deposited under accession number KCTC14149BP as an active ingredient.

In the food composition according to the present invention, each term has the same meaning as described in the pharmaceutical composition unless otherwise specified.

The effective amount of the *Lactobacillus delbrueckii* subsp. *lactis* strain included in the food composition of the present invention may be appropriately determined depending on the intended use (prevention, improvement, or therapeutic treatment). Generally, during the manufacture of food, the *Lactobacillus delbrueckii* subsp. *lactis* strain may be included in the food composition in an amount of 0.001 to 20% by weight, 0.001 to 15% by weight, or 0.001 to 10% by weight. In the case of health beverages, based on 100 mL, it may be included in an amount of 0.01 to 2 g, specifically 0.02 to 2 g, and more specifically 0.3 to 1 g. However, in the case of long-term intake for the purpose of health maintenance, hygiene, or health regulation, it may be used in an amount below the above range. In the process of manufacturing the food composition of the present invention, the content of the *Lactobacillus delbrueckii* subsp. *lactis* strain added to the food composition may be appropriately increased or decreased as needed.

In addition, the food composition may include, as active ingredients, not only the lactic acid bacteria but also ingredients that are conventionally added during food manufacturing. The additional ingredients include, for example, proteins, carbohydrates, fats, nutrients, seasoning agents, and flavoring agents. The carbohydrates may include monosaccharides (for example, glucose, fructose, etc.), disaccharides (for example, maltose, sucrose, oligosaccharides, etc.), and polysaccharides (for example, typical sugars such as dextrin, cyclodextrin, and sugar alcohols such as xylitol, sorbitol, erythritol). As flavoring agents, natural flavoring agents (for example, thaumatin, stevia extract such as rebaudioside A, glycyrrhizin, etc.) and synthetic flavoring agents (for example, saccharin, aspartame, etc.) may be used.

For example, when the food composition of the present invention is manufactured as a drink, it may additionally include citric acid, liquid fructose, sugar, glucose, acetic acid, malic acid, fruit juice, jujube extract, or licorice extract, in addition to the strain which is the active ingredient of the present invention.

The food composition of the present invention includes all processed forms of natural materials such as food, functional food, nutritional supplement, health food, and food additives. The types of food compositions may be manufactured in various forms according to conventional methods known in the art, and during such manufacturing, ingredients and materials conventionally added in the art may be added.

For example, as health foods, the lactic acid bacteria may be manufactured in the form of tea, juice, and drinks for drinking, or may be granulated, encapsulated, or powdered for intake. Additionally, as foods, the lactic acid bacteria of the present invention may be added and manufactured into beverages including alcoholic beverages, fruits and their processed foods (e.g., canned fruits, bottled fruits, jams, marmalades, etc.), fish, meat and their processed foods (e.g., ham, sausages, corned beef, etc.), breads and noodles (e.g., udon, buckwheat noodles, ramen, spaghetti, macaroni, etc.), fruit juices, various drinks, cookies, taffy, dairy products (e.g., yogurt, fermented milk, butter, cheese, etc.), edible vegetable oils, margarine, vegetable proteins, retort foods, frozen foods, and various seasonings (e.g., soybean paste, soy sauce, sauces, etc.). In addition, to use the lactic acid bacteria of the present invention in the form of food additives, it may be manufactured into the form of pills, powders, or concentrates.

Moreover, unlike general pharmaceuticals, food has the advantage of being free from side effects that may occur from long-term administration of drugs and offers excellent portability. Accordingly, the food composition of the present invention can be used as an adjuvant to enhance or improve the preventive or therapeutic effects on cognitive impairment or Alzheimer's disease (AD), and may also be used simultaneously or sequentially with the pharmaceutical composition according to the present invention and/or other compositions or therapies to maximize the above effects.

The present invention provides a composition for the prevention, improvement, or treatment of cognitive impairment or Alzheimer's disease (AD), comprising a *Lactobacillus delbrueckii* subsp. *lactis* strain as an active ingredient. The strain of the present invention has excellent efficacy in reducing amyloid beta protein (Aβ) or tau protein (Tau) and improving cognitive function, and thus can be usefully employed as a composition for the prevention, improvement, or treatment of cognitive impairment or AD.

### [Brief Description of Figures]

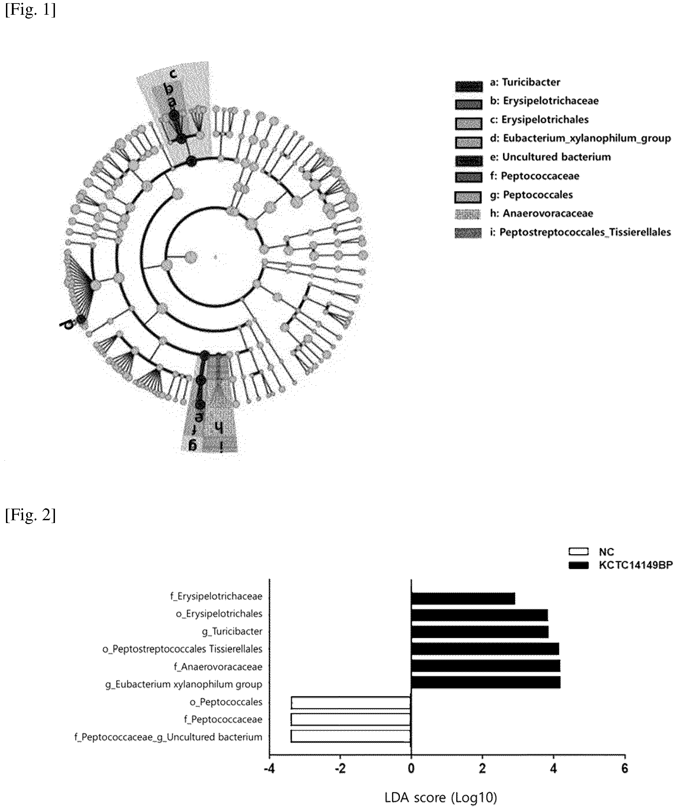

FIG. 1 is a cladogram showing changes in the gut microbiota after administering the *Lactobacillus delbrueckii* subsp. *lactis* strain of the present invention to an Alzheimer's disease (AD) animal model. Green indicates increased bacteria, and red indicates decreased bacteria.
FIG. 2 is a plot showing changes in the gut microbiota after administering *Lactobacillus delbrueckii* subsp. *lactis* to an AD animal model. NC refers to PBS-administered ADLP^{APT} mice, and KCTC14149BP refers to *Lactobacillus delbrueckii* subsp. lactis-administered ADLP^{APT} mice.

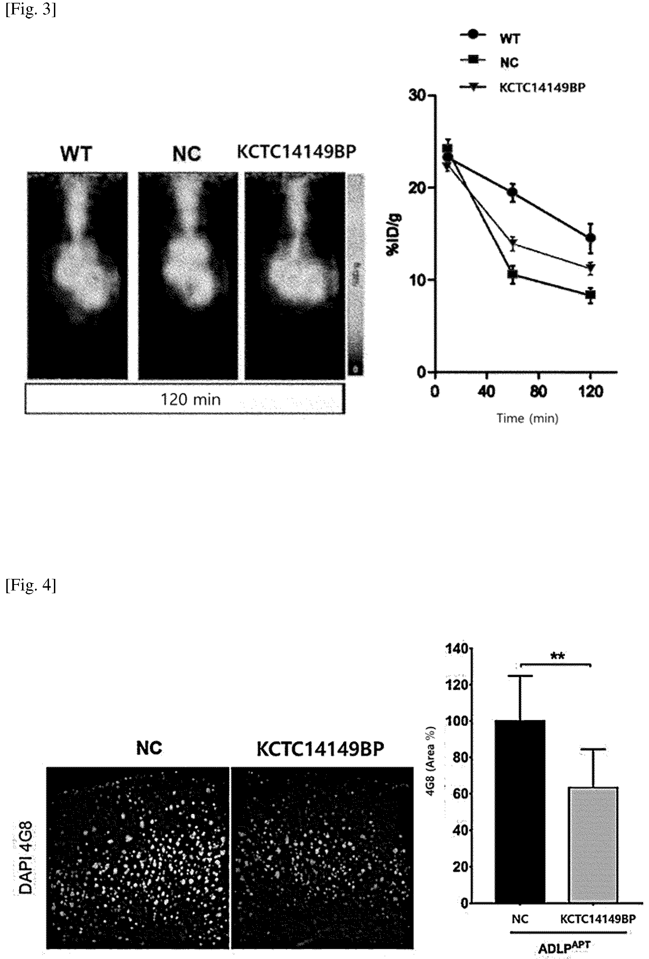

FIG. 3 is an image and graph showing the intestinal barrier permeability results of experimental animals after administering *Lactobacillus delbrueckii* subsp. *lactis* to an AD animal model. WT refers to PBS-administered ADLP^{WT} mice, NC refers to PBS-administered ADLP^{APT} mice, and KCTC14149BP refers to *Lactobacillus delbrueckii* subsp. *lactis-*administered ADLP^{APT} mice.
FIG. 4 is a photograph and graph showing the immunostaining results for amyloid beta protein (Aβ) in brain tissue after administering *Lactobacillus delbrueckii* subsp. *lactis* to an AD animal model. In the immunostaining photographs, blue indicates cell nuclei stained with DAPI, and green indicates Aβ plaques bound with the primary antibody. NC refers to PBS-administered ADLP^{APT} mice, KCTC14149BP refers to *Lactobacillus delbrueckii* subsp. *lactis-*administered ADLP^{APT} mice, and 4G8 refers to the primary antibody Biotin-labeled 4G8.

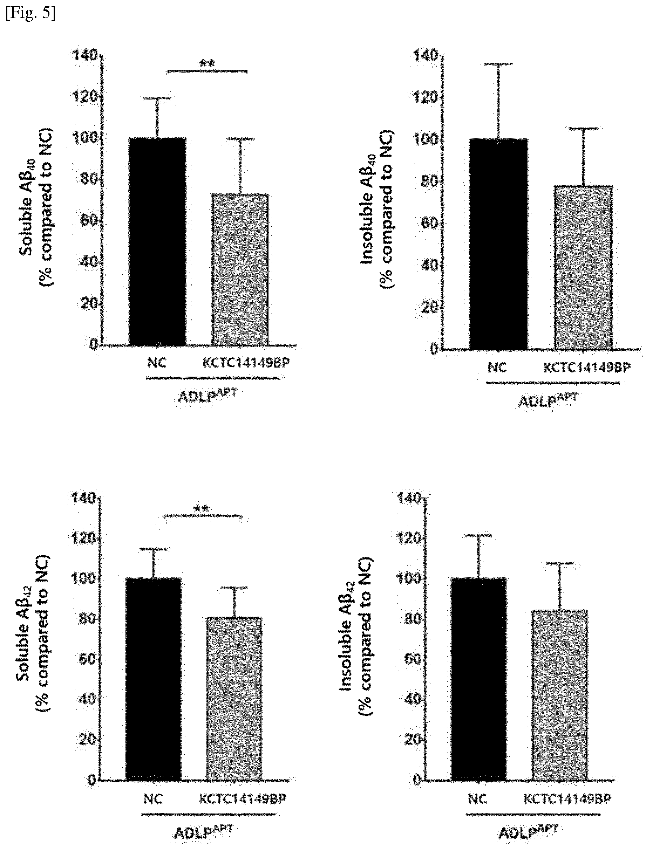

FIG. 5 is a graph showing the ELISA results of Aβ in brain tissue after administration of *Lactobacillus delbrueckii* subsp. *lactis* to an AD animal model. NC refers to PBS-administered ADLP^{APT} mice, KCTC14149BP refers to *Lactobacillus delbrueckii* subsp. lactis-administered ADLP^{APT} mice, and ** indicates P value < 0.01.

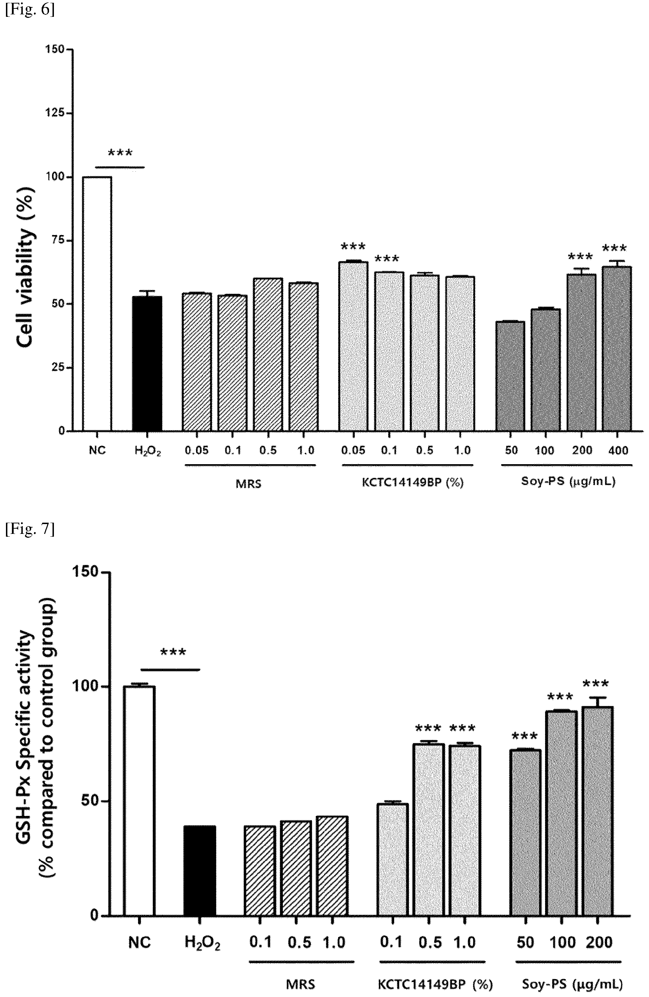

FIG. 6 is a graph showing the neuroprotective ability of the strain. NC refers to the control group, H₂O₂ refers to the H₂O₂-treated control group, MRS refers to the MRS-treated group, KCTC14149BP refers to the *Lactobacillus delbrueckii* subsp. *lactis-treated* group, Soy-PS refers to the Soy-PS-treated group, and *** indicates P value < 0.001.
FIG. 7 is a result showing the antioxidant activity of *Lactobacillus delbrueckii* subsp. *lactis,* and is a graph showing the percentage (%) compared to the control group (NC) after measuring the amount of glutathione in nerve cells. NC refers to the control group, H₂O₂ refers to the H₂O₂-treated control group, MRS refers to the MRS-treated group, KCTC14149BP refers to the *Lactobacillus delbrueckii* subsp. *lactis-treated* group, Soy-PS refers to the Soy-PS-treated group, and *** indicates P value < 0.001.

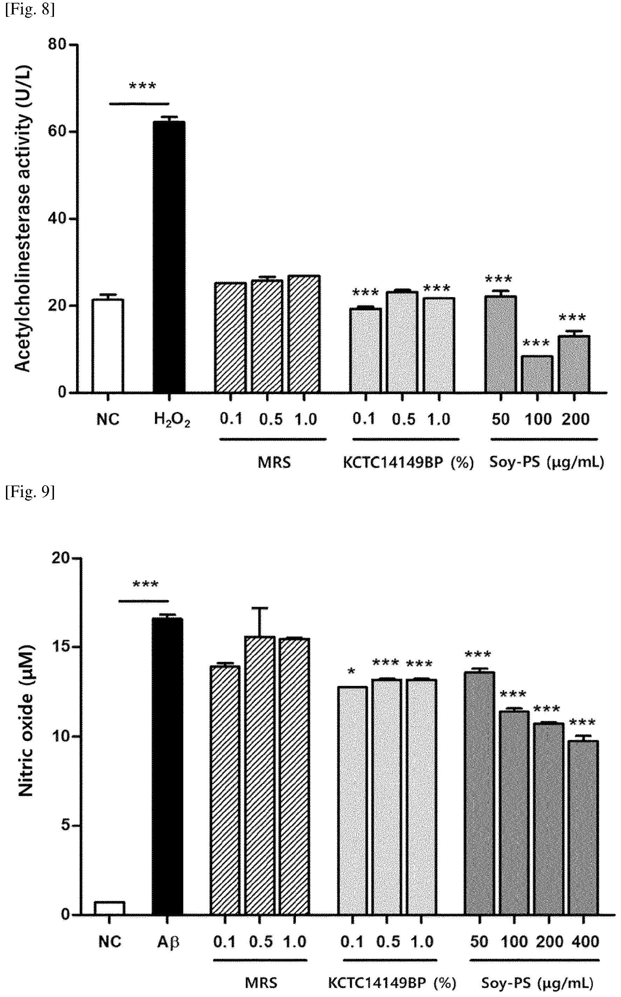

FIG. 8 is a graph showing the acetylcholinesterase (AChE) activity of Lactobacillus *delbrueckii* subsp. *lactis.* NC refers to the control group, H₂O₂ refers to the H₂O₂-treated control group, MRS refers to the MRS-treated group, KCTC14149BP refers to the *Lactobacillus delbrueckii* subsp. *lactis-treated* group, Soy-PS refers to the Soy-PS-treated group, and *** indicates P value < 0.001.
FIG. 9 is a result showing the anti-inflammatory activity of *Lactobacillus delbrueckii* subsp. *lactis,* and is a graph showing the amount of nitric oxide (NO) in microglial cells. NC refers to the control group, Aβ refers to the Aβ₁₋₄₂-treated control group, MRS refers to the MRS-treated group, KCTC14149BP refers to the *Lactobacillus delbrueckii* subsp. *lactis-treated* group, Soy-PS refers to the Soy-PS-treated group, * indicates P value < 0.05, and *** indicates P value < 0.001.

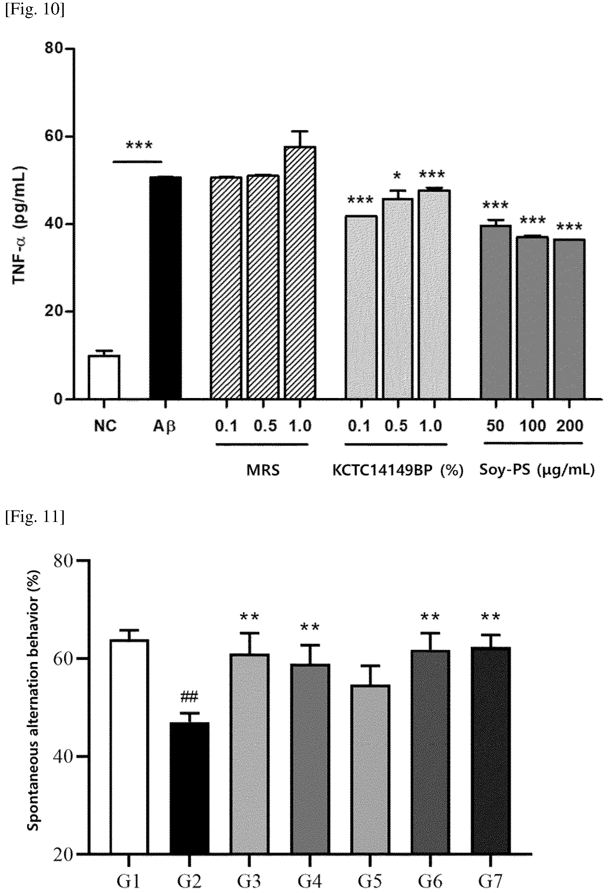

FIG. 10 is a result showing the anti-inflammatory activity of *Lactobacillus delbrueckii* subsp. *lactis,* and is a graph showing the amount of tumor necrosis factor-α (TNF-α) in microglial cells. NC refers to the control group, Aβ refers to the Aβ₁₋₄₂-treated control group, MRS refers to the MRS-treated group, KCTC14149BP refers to the *Lactobacillus delbrueckii* subsp. *lactis-treated* group, Soy-PS refers to the Soy-PS-treated group, * indicates P value < 0.05, and *** indicates P value < 0.001.
FIG. 11 is a graph showing the effect of improving short-term memory ability and spatial perception ability of experimental animals through a Y-maze test after administration of *Lactobacillus delbrueckii* subsp. *lactis* to a scopolamine-induced cognitive impairment animal model. G1 refers to the control group, G2 refers to the vehicle-treated group, G3 refers to the donepezil-treated group, G4 refers to the decursin-treated group, G5 refers to the low-dose *Lactobacillus delbrueckii* subsp. *lactis-treated* group, G6 refers to the medium-dose *Lactobacillus delbrueckii* subsp. *lactis-treated* group, G7 refers to the high-dose *Lactobacillus delbrueckii* subsp. *lactis-treated* group, ## indicates P value < 0.01 against G1, and ** indicates P value < 0.01 against G2.

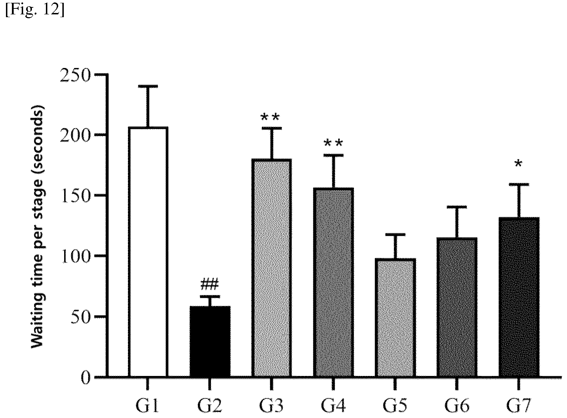

FIG. 12 is a graph showing the effect of improving cognitive ability of experimental animals through a passive avoidance test after administration of *Lactobacillus delbrueckii* subsp. *lactis* to a scopolamine-induced cognitive impairment animal model. G1 is the control group, G2 is the vehicle-treated group, G3 is the donepezil-treated group, G4 is the decursin-treated group, G5 is the low-dose *Lactobacillus delbrueckii* subsp. *lactis-treated* group, G6 is the medium-dose *Lactobacillus delbrueckii* subsp. *lactis-treated* group, G7 is the high-dose *Lactobacillus delbrueckii* subsp. *lactis-treated* group, ## indicates P value < 0.01 against G1, * indicates P value < 0.05 against G2, and ** indicates P value < 0.01 against G2.

### [Examples]

Hereinafter, the present invention will be described in more detail through examples. These examples are provided solely for the purpose of more specifically describing the present invention, and it will be apparent to those skilled in the art that the scope of the present invention is not limited by these examples according to the gist of the present invention. In this specification, unless otherwise specifically stated, the singular form also includes the plural form.

Throughout the present specification, "%" used to indicate the concentration of a specific substance refers to (weight/weight)% for solid/solid, (weight/volume)% for solid/liquid, and (volume/volume)% for liquid/liquid, unless otherwise specified.

### Preparation Example 1: Preparation of Probiotics

*Lactobacillus delbrueckii* subsp. *lactis* used in this experiment was isolated from raw milk fermentation products. Lactic acid bacteria starter culture was performed in a flask containing MRS Broth at 37°C for 24 hours. The starter culture was inoculated into an optimized self-produced medium. The culture was carried out at 37°C for 18 to 20 hours under the following conditions: the pH was maintained at 5.5 to 6.0 and agitation was performed at 55 to 65 RPM. Freeze-drying of 40X concentrated cells was performed according to the manual. After freeze-drying, the colony-forming unit (CFU) per 1g of each probiotic powder was measured by the serial dilution method. The probiotics were suspended in 1X PBS and adjusted to a density of 5 × 10⁹ CFU/200µL before use.

### Preparation Example 2: Preparation of Alzheimer's Disease (AD) Animal Model

The transgenic ADLP (Alzheimer's disease-like pathology) animal model was prepared as follows. i) 5XFAD mice (Tg6977, Jackson Laboratory, Stock #006554), which express the human amyloid precursor protein (APP) gene carrying the Swedish (K670N/M671L), Florida (I716V), and London (V717I) mutations and the human presenilin-1 (PSEN1) gene carrying the M146L and L286V mutations under the control of the Thy1 promoter, were crossed with ii) JNPL3 mice (TauP301L-JNPL3, Taconic, Stock #2508 homozygote), which express the human Tau gene carrying the P301L mutation under the control of the prion protein promoter.

Because the ADLP^{APT} mice, which express a total of three mutant human genes, exhibit earlier pathological changes of Alzheimer's disease and cognitive impairment in females than in males, only female mice were used in this experiment.

### Example 1: Changes in Gut Microbiota Composition

In order to determine whether the strain according to the present invention can prevent, alleviate, or treat cognitive impairment or AD by changing the gut microbiota, changes in the gut microbiota composition of the AD animal model by oral administration of the strain were analyzed.

Specifically, 2- to 2.5-month-old ADLPAPT mice were administered either a PBS suspension of *Lactobacillus delbrueckii* subsp. *lactis* (strain-treated group) or PBS alone (negative control, NC) at a volume of 200 µL. The normal control group (wild type, WT) consisting of ADLP^{WT} mice was administered 200 µL of PBS. Administration was performed orally five times per week for a period of five months.

After completion of administration, fecal samples were collected before the sacrifice of the experimental animals and stored at -80°C until analysis. Genomic DNA was extracted from the fecal samples using the QIAamp DNA Stool Mini Kit (Qiagen, 51304). The V3-V4 region of the 16S rRNA gene was amplified by PCR using primers 341F (5'-CCTACGGGNGGCWGCAG-3') and 805R (5'-GACTACHVGGGTATCTAATCC-3'). The amplified PCR products were purified using HiAccuBead (AccuGene, ACN01.50). Metagenome sequencing was performed using the Ion Torrent S5 Sequencer System.

The generated sequences were analyzed using the QIIME2 pipeline (version 2022.2) to identify the overall genetic information of the gut microbiota. In order to confirm differences in microbial genera between the strain administration group and the NC group, the Linear Discriminant Analysis (LDA) Effect Size (LEfSe) algorithm was used at the genus level. As shown in FIGS. 1 and 2, in the strain administration group, the intestinal distribution of *Erysipelotrichaceae, Erysipelotrichales, Turicibacter, Peptostreptococcales Tissierellales, Anaerovoracaceae,* and *Eubacterium xylanophilum* increased, whereas the intestinal distribution of *Peptoc Peptococcales* and *Peptococaceae* decreased.

### Example 2: Decrease in Intestinal Permeability

Patients with cognitive impairment or AD exhibit a "leaky gut" condition in which the intestinal barrier function is impaired and intestinal permeability is decreased, thereby activating systemic inflammatory responses and leading to cognitive decline (Vanessa Stadlbauer et al., BMC Geriatrics 20 (248), 20 July 2020).

Accordingly, in order to evaluate whether the strain according to the present invention can prevent, alleviate, or treat cognitive impairment or AD by improving the impaired intestinal barrier function, changes in intestinal permeability were assessed in an AD animal model after oral administration of the strain.

Specifically, the strain was administered to the AD animal model according to the method of Example 1. After completion of administration, the animals were fasted for 4 hours before the intestinal permeability evaluation, and 0.6 mg/g of a 4 kDa fluorescent substance was administered orally. Two hours after the administration of the fluorescent substance, the materials present in the intestine were visualized and the residual amount was measured to evaluate the intestinal permeability.

As a result, as shown in FIG. 3, it was confirmed that the amount of fluorescent substance remaining in the intestine of the ADLP^{APT} mice (NC) was significantly decreased compared to the ADLP^{WT} mice (WT). In the strain administration group, the residual amount of fluorescent substance in the intestine increased compared to NC. These results suggest that the *Lactobacillus delbrueckii* subsp. *lactis* strain of the present invention can exert an effect of preventing, alleviating, or treating cognitive impairment or AD by reducing the increased intestinal permeability.

### Example 3: Reduction of Amyloid beta Protein (Aβ)

Cognitive impairment or AD is known to develop due to the accumulation of Aβ. Accordingly, in order to evaluate whether the strain according to the present invention can prevent, alleviate, or treat cognitive impairment or AD, changes in brain Aβ plaques in the AD animal model by oral administration of the strain were assessed by immunohistochemistry and enzyme-linked immunosorbent assay (ELISA).

### 3-1. Immunohistochemistry

Specifically, the strain was administered to the AD animal model according to the method of Example 1. After completion of administration, the experimental animals were anesthetized with a mixture of Tiletamine-Zolazepam and Xylazine (1.2 mg/kg) and perfused with PBS. The brain tissues of the experimental animals were fixed in 4% paraformaldehyde for 24 hours and then dehydrated in a 30% sucrose solution for about 72 hours for immunofluorescence staining. The pretreated brain tissues were frozen at -80°C and then coronally sectioned using a microtome capable of maintaining -25°C. The brain tissue sections were washed with PBS, subjected to blocking and permeabilization, and then reacted with primary antibodies Biotin-labeled 4G8 (1:700, Covance, SIG-39240), GFAP (1:1,000, Invitrogen, 13-0300), and Iba1 (1:500, Wako, 019-19741). The brain tissue sections reacted with the primary antibodies were washed with PBS and further reacted with fluorescently labeled secondary antibodies, followed by DAPI staining and observation under a confocal microscope. As a result, as shown in FIG. 4, the area occupied by Aβ plaques in the brain was significantly reduced by about 30% in the strain administration group compared to the control group (NC).

These results suggest that the *Lactobacillus delbrueckii* subsp. *lactis* strain of the present invention can exert an effect of preventing, alleviating, or treating cognitive impairment or Alzheimer's disease by removing Aβ plaques accumulated in the brain.

### 3-2. ELISA

Specifically, the strain was administered to the AD animal model according to the method of Example 1. After completion of administration, the experimental animals were anesthetized with a mixture of Tiletamine-Zolazepam and Xylazine (1.2mg/kg) and perfused with PBS. The brain tissues of the experimental animals were homogenized in RIPA buffer (50mM Tris-HCL, pH 7.4; 150mM NaCl; 1% Nonidet P-40; 0.1% SDS; 0.5% deoxycholic acid sodium salt) for ELISA experiments, and the protein concentration in the supernatant of the tissue extract was quantified. 100µg of protein was ultracentrifuged to separate the RIPA-soluble and RIPA-insoluble fractions. The RIPA-insoluble fraction was re-suspended in 70% formic acid and neutralized with 1M Tris-base solution before the ELISA experiment. The concentrations of RIPA-soluble Aβ₄₀ and Aβ₄₂, as well as RIPA-insoluble Aβ₄₀ and Aβ₄₂, were measured according to the protocol of the human Aβ-specific ELISA kit.

As a result, as shown in FIG. 5, the levels of RIPA-soluble Aβ₄₀ and Aβ₄₂ in the brain were significantly reduced by approximately 27% and 19%, respectively, in the strain administration group compared to the control group (NC). In addition, the levels of RIPA-insoluble Aβ₄₀ and Aβ₄₂ were reduced by approximately 22% and 16%, respectively.

These results suggest that the *Lactobacillus delbrueckii* subsp. *lactis* strain of the present invention can exert an effect of preventing, ameliorating, or treating cognitive impairment or Alzheimer's disease by reducing soluble or insoluble forms of Aβ present in the brain.

### Example 4: Neuroprotective Effect

In order to evaluate whether the strain according to the present invention can prevent, improve, or treat cognitive impairment or AD, the effect of the strain on neuroprotection, as represented by the reduction of reactive oxygen species (ROS), was assessed.

Hydrogen peroxide (H₂O₂), a ROS, was added to nerve cells, followed by an MTT assay. ROS such as H₂O₂ are associated with various biological processes and especially regulate cellular differentiation, gene expression, and responses to cytokines. Therefore, maintaining the homeostasis of ROS is crucial for cell growth and survival (Sang Won Kang, Hanyang Med. Rev. 2013; 33:77-82).

Specifically, PC-12 nerve cell line was seeded at a concentration of 1.0 x 10⁴ cells/well in a 96-well plate and cultured for 24 hours. H₂O₂, the strain, and Soy-PS were selectively added to the respective wells depending on whether they belonged to the control group or the experimental group. The control and experimental groups were classified as follows. Soy-PS, approved by the Korean Ministry of Food and Drug Safety as a functional ingredient for brain health, is a major phospholipid that forms brain cell membranes and is known to help prevent dementia and improve memory, cognitive function, learning ability, and attention deficit disorders (ADHD), and was therefore used as a positive control.
(1) Control group (NC): No addition of H₂O₂, strain, or Soy-PS
(2) H₂O₂-treated control group (H₂O₂): Addition of 800µM H₂O₂
(3) MRS broth-treated group (MRS): Addition of MRS broth in amounts of 0.05mL, 0.1mL, 0.5mL, and 1.0mL together with 800µM H₂O₂
(4) Strain-treated group (KCTC14149BP): Addition of the strain at concentrations of 0.05%, 0.1%, 0.5%, and 1.0% together with 800µM H₂O₂
(5) Soy-PS-treated group (Soy-PS): Addition of Soy-PS at concentrations of 50µg/mL, 100µg/mL, 200µg/mL, and 400µg/mL together with 800µM H₂O₂

After adding the substances as described above and reacting for 24 hours, MTT solution was added to each well and reacted for 4 hours. Subsequently, 100µL of DMSO was added to each well to dissolve the purple formazan, and absorbance was measured at 570nm using a spectrophotometer to determine cell viability (%). Cell viability was expressed as a percentage compared to the control group (NC). The experiment was repeated three times and statistically analyzed (n=3 for experimental groups).

As shown in FIG. 6, in PC-12 cells, the strain-treated group (KCTC14149BP) exhibited a significant cell-protective effect compared to the H₂O₂-treated control group, showing approximately a 1.3-fold increase at 0.05% and a 1.2-fold increase at 0.1%. The Soy-PS-treated group (Soy-PS) showed approximately a 1.2-fold increase in cell viability at concentrations of 200 µg/mL and 400 µg/mL compared to the H₂O₂-treated control group, which was comparable to the results observed in the strain-treated group (KCTC14149BP) at 0.05% and 0.1%.

These results suggest that the Lactobacillus delbrueckii subsp. lactis strain of the present invention increases neuronal cell viability and exhibits neuroprotective effects. Since this effect is similar to that of the previously used brain health functional ingredient Soy-PS, it suggests that the strain can have a preventive, improving, or therapeutic effect on cognitive impairment or Alzheimer's disease.

### Example 5: Antioxidant Activity

In order to evaluate whether the strain according to the present invention can prevent, improve, or treat cognitive impairment or AD, the effect of the strain on antioxidant activity was assessed. Specifically, glutathione peroxidase (GSH-Px) activity in nerve cells treated with H₂O₂ as ROS was measured by quantifying the amount of glutathione (GSH). GSH is a component of the antioxidant enzyme GSH-Px, which reduces H₂O₂ to water (H₂O), thereby preventing DNA damage and inducing apoptosis of abnormal cells, indicating antioxidant activity.

Specifically, PC-12 nerve cell line was seeded at a concentration of 1.0 x 10⁴ cells/well in a 96-well plate and cultured for 24 hours. H₂O₂, the strain, and Soy-PS were selectively added to the respective wells depending on whether they belonged to the control group or the experimental group. The control and experimental groups were classified as follows:
(1) Control group (NC): No addition of H₂O₂, strain, or Soy-PS
(2) H₂O₂-treated control group (H₂O₂): Addition of 800µM H₂O₂
(3) MRS broth-treated group (MRS): Addition of MRS broth in amounts of 0.1mL, 0.5mL, and 1.0mL together with 800µM H₂O₂
(4) Strain-treated group (KCTC14149BP): Addition of the strain at concentrations of 0.1 %, 0.5%, and 1.0% together with 800µM H₂O₂
(5) Soy-PS-treated group (Soy-PS): Addition of Soy-PS at concentrations of 50µg/mL, 100µg/mL, and 200µg/mL together with 800µM H₂O₂

After adding the substances as described above and reacting for 24 hours, the control and experimental groups were washed twice with PBS (pH 7.4) and then homogenized in RIPA buffer. The RIPA buffer includes 150mM NaCl, 0.5% Triton X-100, 50mM Tris-HCl, pH 7.4, 25 mM NaF, 20mM EGTA, 1mM DTT, 1mM Na₃VO₄, and protease inhibitor cocktail. After homogenization, the mixture was centrifuged at 1,890 x g for 10 minutes at 4°C to remove the cell supernatant. After adding 400 µL of glutathione assay buffer, the solution was centrifuged at 12,000 × g for 30 minutes at 36°C to obtain the cell supernatant, which was used as the sample.

For the glutathione analysis, a total of 160 µL of glutathione reaction buffer, glutathione reductase, and generation mix (BioVision) was added to a 96-well plate and incubated at room temperature for 10 minutes. Subsequently, 20 µL of either a standard solution or a sample (cell supernatant) was added to each well and reacted again for 10 minutes at room temperature. Then, 20 µL of substrate solution was added to induce color development, and the absorbance was measured at 405 nm using a spectrophotometer. The GSH concentration in the sample was calculated from the standard calibration curve and expressed as a percentage (%) relative to the control group (NC). The experiment was performed in triplicate and subjected to statistical analysis (n = 3 per experimental group).

As a result, as shown in FIG. 7, the GSH amount in PC-12 cells was significantly increased by about 1.9 times at concentrations of 0.5% and 1.0% in the strain-treated group (KCTC14149BP) compared to the H₂O₂-treated control group. Specifically, the strain-treated group (KCTC14149BP) exhibited about 84% of the GSH amount of the Soy-PS-treated group (Soy-PS), indicating that the strain of the present invention and Soy-PS exhibited similar antioxidant activity.

These results suggest that the *Lactobacillus delbrueckii* subsp. *lactis* strain of the present invention increases antioxidant activity and demonstrates neuroprotective effects. Since these effects are similar to those of Soy-PS, a previously used brain health functional ingredient, they suggest that the strain can have a preventive, improving, or therapeutic effect on cognitive impairment or Alzheimer's disease.

### Example 6: Inhibition of Acetylcholinesterase (AChE)

In order to evaluate whether the strain according to the present invention can prevent, improve, or treat cognitive impairment or AD, the effect of the strain on acetylcholinesterase (AChE) activity inhibition was assessed.

Specifically, AChE activity was induced by hydrogen peroxide (H₂O₂), and the level of AChE activity was measured for each control and experimental group. PBS was added to each well of a 96-well plate at 150µL, and H₂O₂, the strain, and Soy-PS were selectively added to the control and experimental groups. The control and experimental groups were classified as follows:
(1) Control group (NC): No addition of H₂O₂, strain, or Soy-PS, but 0.2U/mL of AChE was added.
(2) H₂O₂-treated control group (H₂O₂): Addition of 800µM H₂O₂ and 0.2U/mL of AChE.
(3) MRS broth-treated group (MRS): Addition of various amounts of MRS broth (0.1mL, 0.5mL, 1.0mL) together with 800µM H₂O₂ and 0.2U/mL of AChE.
(4) Strain-treated group (KCTC14149BP): Addition of the strain at concentrations of 0.1 %, 0.5%, and 1.0% together with 800µM H₂O₂ and 0.2U/mL of AChE.
(5) Soy-PS-treated group (Soy-PS): Addition of Soy-PS at concentrations of 50µg/mL, 100µg/mL, and 200µg/mL together with 800µM H₂O₂ and 0.2U/mL of AChE.

After adding the substances as described above, the samples were kept at 37°C for 5 minutes. Then, 30µL of 10mM DTNB and 20µL of 15mM ATCI were added, followed by incubation at 37°C for 30 minutes. The absorbance was measured at 415nm using a fluorescence spectrophotometer. AChE inhibition activity was expressed in nM tacrine equivalents, and the experiment was repeated three times and statistically analyzed (n=3 for experimental groups).

As a result, as shown in FIG. 8, the strain-treated group (KCTC14149BP) showed a significant decrease in AChE activity compared to the H₂O₂-treated control group, with a reduction of approximately 70% at concentrations of 0.1% and 1.0%, and about 60% at 0.5%. Specifically, the Soy-PS-treated group (Soy-PS) showed about a 70% decrease at 50 µg/mL, about 90% at 100 µg/mL, and about 80% at 200 µg/mL compared to the H₂O₂-treated control group, which was similar to the results observed in the strain-treated group (KCTC14149BP), confirming that the strain of the present invention and Soy-PS exhibited similar effects in inhibiting AChE activity.

These results suggest that the *Lactobacillus delbrueckii* subsp. *lactis* strain of the present invention inhibits AChE activity, demonstrating neuroprotective effects. Since these effects are similar to those of Soy-PS, a previously used brain health functional ingredient, it suggests that the strain can have a preventive, improving, or therapeutic effect on cognitive impairment or Alzheimer's disease.

### Example 7: Anti-inflammatory Activity

In order to evaluate whether the strain according to the present invention can prevent, improve, or treat cognitive impairment or AD, the effect of the strain on anti-inflammatory activity was assessed. The deposition of Aβ in the brain is associated with an inflammatory response, and it is mediated by the activation of microglial cells, which are immune cells that surround the Aβ plaques. Microglial cells are activated through phagocytosis, an immune response, to remove Aβ, and they play a critical role in brain inflammation by readily responding to signals from the extracellular environment. Aβ-activated microglial cells produce inflammatory mediators such as cyclooxygenase-2 (COX-2), inducible Nitric oxide synthase (iNOS), and increase the synthesis and secretion of inflammatory cytokines such as Interleukin-1 beta (IL-1β), Interleukin-6 (IL-6), or tumor necrosis factor-α (TNF-α), and thus regulation of their activation is required. Previous studies have shown that regulating this inflammatory process with long-term use of anti-inflammatory agents can suppress and improve the progression of AD (Hui-Jin Mun et al., JKSCI, Vol. 25 No. 6, 165-170).

### Example 7-1: Reduction of Nitric oxide (NO)

Specifically, the amount of nitric oxide (NO) in microglial cells upon Aβ-induced inflammation was measured.

BV-2 microglial cells were seeded at a concentration of 1.0 x 10⁴ cells/well in a 96-well plate and cultured for 24 hours. Aβ₁₋₄₂, the strain, and Soy-PS were selectively added to the control and experimental groups in the wells. The control and experimental groups were classified as follows:
(1) Control group (NC): No addition of Aβ₁₋₄₂, strain, or Soy-PS
(2) Aβ1-42-treated control group (Aβ): Addition of 20µM Aβ1-42
(3) MRS broth-treated group (MRS): Addition of various amounts of MRS broth (0.1mL, 0.5mL, 1.0mL) together with 20µM Aβ₁₋₄₂
(4) Strain-treated group (KCTC14149BP): Addition of the strain at concentrations of 0.1 %, 0.5%, and 1.0% together with 20µM Aβ₁₋₄₂
(5) Soy-PS-treated group (Soy-PS): Addition of Soy-PS at concentrations of 50µg/mL, 100µg/mL, 200µg/mL, and 400µg/mL together with 20µM Aβ₁₋₄₂

After adding the substances to the control and experimental groups and reacting for 24 hours, the cell culture was collected, and the amount of NO was measured using the Griess Reagent System. The NO amount was converted based on the sodium nitrite standard curve, and the experiment was repeated three times and statistically analyzed (n=3 for experimental groups).

As a result, as shown in FIG. 9, the amount of NO in BV-2 cells was decreased in the strain-treated group (KCTC14149BP) by about 20% at concentrations of 0.1%, 0.5%, and 1.0% compared to the Aβ₁₋₄₂-treated control group (Aβ). Specifically, the Soy-PS-treated group (Soy-PS) showed a reduction of about 10% at 50 µg/mL, about 30% at both 100 µg/mL and 200 µg/mL, and about 40% at 400 µg/mL compared to the Aβ₁₋₄₂-treated control group (Aβ), showing a similar level of reduction to that observed in the strain-treated group (KCTC14149BP).

These results suggest that the *Lactobacillus delbrueckii* subsp. *lactis* strain of the present invention reduces NO levels, demonstrating anti-inflammatory activity. Since this effect is similar to that of Soy-PS, a previously used brain health functional ingredient, it suggests that the strain can have a preventive, improving, or therapeutic effect on cognitive impairment or Alzheimer's disease.

### Example 7-2: Reduction of Inflammatory Cytokines

Specifically, the amount of the inflammatory cytokine TNF-α in microglial cells upon Aβ-induced inflammation was measured.

BV-2 microglial cells were seeded at a concentration of 1.0 x 10⁴ cells/well in a 96-well plate and cultured for 24 hours. Aβ₁₋₄₂, the strain, and Soy-PS were selectively added to the control and experimental groups in the wells. The control and experimental groups were classified as follows:
(1) Control group (NC): No addition of Aβ₁₋₄₂, strain, or Soy-PS
(2) Aβ₁₋₄₂-treated control group (Aβ): Addition of 20µM Aβ₁₋₄₂
(3) MRS broth-treated group (MRS): Addition of various amounts of MRS broth (0.1mL, 0.5mL, 1.0mL) together with 20µM Aβ₁₋₄₂
(4) Strain-treated group (KCTC14149BP): Addition of the strain at concentrations of 0.1 %, 0.5%, and 1.0% together with 20µM Aβ₁₋₄₂
(5) Soy-PS-treated group (Soy-PS): Addition of Soy-PS at concentrations of 50µg/mL, 100µg/mL, and 200µg/mL together with 20µM Aβ₁₋₄₂

After adding the substances to the control and experimental groups and reacting for 24 hours, the cell culture was collected, and the amount of TNF-α was measured. The amount of TNF-α in the sample (cell culture) was determined from the standard curve. The experiment was repeated three times and statistically analyzed (n=3 for experimental groups).

As a result, as shown in FIG. 10, the amount of TNF-α in BV-2 cells was decreased in the strain-treated group (KCTC14149BP) by about 20% at a concentration of 0.1%, and by about 10% at concentrations of 0.5% and 1.0% compared to the Aβ₁₋₄₂-treated control group (Aβ). Specifically, the Soy-PS-treated group (Soy-PS) showed a decrease of approximately 20% at concentrations of 50 µg/mL, 100 µg/mL, and 200 µg/mL compared to the Aβ₁₋₄₂-treated control group (Aβ), which was similar to the level of TNF-α observed in the strain-treated group (KCTC14149BP), confirming that the strain of the present invention and Soy-PS exhibited comparable effects in reducing TNF-α.

These results suggest that the *Lactobacillus delbrueckii* subsp. *lactis* strain of the present invention reduces TNF-α levels, demonstrating anti-inflammatory activity. Since this effect is similar to that of Soy-PS, a previously used brain health functional ingredient, it suggests that the strain can have a preventive, improving, or therapeutic effect on cognitive impairment or Alzheimer's disease.

### Example 8: Y-Maze Test

In order to evaluate whether the strain according to the present invention can prevent, improve, or treat cognitive impairment or AD, the effect of the strain on short-term memory and momentary spatial perception ability was assessed using the Y-maze test.

The Y-maze test is used to assess short-term memory and momentary spatial perception by evaluating the animal's ability to remember the previously explored arm, with higher memory performance indicated by a lower tendency to re-enter the same arm.

Specifically, scopolamine, which induces memory impairment, was administered together with either saline (vehicle), a known treatment for cognitive impairment or AD (donepezil or decursin), or the strain of the present invention, followed by the Y-maze test.

Scopolamine is a muscarinic receptor antagonist that blocks muscarinic receptors located on the postsynaptic cholinergic neurons. It blocks the binding of acetylcholine, a neurotransmitter released from presynaptic neurons, to muscarinic receptors, and this inhibition of neurotransmission impairs learning and memory. Therefore, scopolamine is widely used in studies evaluating improvements in cognitive functions such as learning and memory (Dae-eok Kim et al., Journal of Oriental Neuropsychiatry, 2018; 29(3):121-134).

Donepezil, an AChE inhibitor, is approved by the FDA for the treatment of AD. Acetylcholine (ACh) is one of the neurotransmitters in the autonomic nervous system, which helps with learning and memory. In AD patients, acetylcholine levels are reduced. Accordingly, AChE inhibitors such as donepezil are prescribed to inhibit AChE, which breaks down ACh into acetate ions and choline.

Decursin is a functional ingredient certified for improving cognitive function. It inhibits AChE in the hippocampal tissue, which is closely related to memory and learning, thereby exerting memory-enhancing effects (Ki Yong Lee et al., Korean Journal of Pharmacognosy, 2008; 39(2):86-90).

The Y-maze consists of three arms with dimensions of 40 cm in length, 3 cm in width, and 15 cm in height, with the angles between the arms set at 120°.

Specifically, the experimental animals were divided into seven groups, categorized as control group (G1) and experimental groups (G2 to G7), with each substance administered at 200µL.
(1) Control group (G1): No administration of scopolamine, donepezil, decursin, or the strain
(2) Vehicle-treated group (G2): Administration of scopolamine and vehicle
(3) Donepezil-treated group (G3): Administration of scopolamine and 5mg/kg donepezil
(4) Decursin-treated group (G4): Administration of scopolamine and 5mg/kg decursin
(5) Low-dose strain-treated group (G5): Administration of scopolamine and 1 x 10⁸ CFU/head strain
(6) Medium-dose strain-treated group (G6): Administration of scopolamine and 5 x 10⁸ CFU/head strain
(7) High-dose strain-treated group (G7): Administration of scopolamine and 1 x 10⁹ CFU/head strain

The control and experimental groups were placed at the center of the Y-maze, where they were allowed to explore the maze for 8 minutes. The movement of each individual was analyzed using EthoVision XT16 (Noldus, USA). The experimental results were obtained as spontaneous alternation behavior (%) and calculated according to the following equation: Spontaneous alternation (%) = (Number of spontaneous alternations / (Total number of arm entries - 2)) x 100

The number of spontaneous alternations refers to the number of times that the animal consecutively entered three different arms, and the total arm entries refers to the total number of times that the experimental animal entered any of the arms. The experiment was conducted with 10 animals per group, followed by statistical analysis (n = 10 for experimental groups).

As a result, as shown in FIG. 11, the short-term memory ability and momentary spatial perception ability were assessed, and the low-dose strain-treated group (G5), medium-dose strain-treated group (G6), and high-dose strain-treated group (G7) showed a concentration-dependent increase. Additionally, compared to the vehicle-treated group (G2), the medium-dose strain-treated group (G6) showed about a 1.3-fold increase, and the high-dose strain-treated group (G7) showed about a 1.4-fold increase. Specifically, the donepezil-treated group (G3) and decursin-treated group (G4) showed about a 1.3-fold increase compared to the vehicle-treated group (G2), which was similar to the results in the medium-dose (G6) and high-dose (G7) strain-treated groups.

These results suggest that the Lactobacillus delbrueckii subsp. lactis strain of the present invention improves short-term memory and instant spatial perception abilities, and these effects are similar to those of donepezil or decursin, which are cognitive impairment or AD therapeutic agents. This implies that the strain can have a preventive, improving, or therapeutic effect on cognitive impairment or Alzheimer's disease.

### Example 9. Passive avoidance test

In order to evaluate whether the strain according to the present invention can prevent, improve, or treat cognitive impairment or AD, the effect of the strain on cognitive ability was assessed through the passive avoidance test.

The passive avoidance test is a cognitive ability assessment experiment that utilizes the preference of rodents to stay in dark places.

Specifically, saline (as a vehicle), donepezil (a therapeutic agent for cognitive impairment or AD), decursin (a functional ingredient recognized for improving cognitive function), or the strain of the present invention was co-administered with scopolamine which induces memory impairment, followed by the passive avoidance test.

The experimental animals were divided into seven groups, categorized as control group (G1) and experimental groups (G2 to G7), with each substance administered at 200µL.
(1) Control group (G1): No administration of scopolamine, donepezil, decursin, or the strain
(2) Vehicle-treated group (G2): Administration of scopolamine and vehicle (saline)
(3) Donepezil-treated group (G3): Administration of scopolamine and 5mg/kg donepezil
(4) Decursin-treated group (G4): Administration of scopolamine and 5mg/kg decursin
(5) Low-dose strain-treated group (G5): Administration of scopolamine and 1 x 10⁸ CFU/head strain
(6) Medium-dose strain-treated group (G6): Administration of scopolamine and 5 x 10⁸ CFU/head strain
(7) High-dose strain-treated group (G7): Administration of scopolamine and 1 x 10⁹ CFU/head strain

The control and experimental groups were placed in chambers consisting of a bright chamber and a dark chamber of the same size. When an animal entered the dark chamber from the bright chamber, a 0.5 mA current was applied for 3 seconds. After 24 hours, the animals were again placed in the bright chamber, and the time taken to enter the dark chamber was measured for up to 300 seconds to evaluate memory.

As a result, as shown in FIG. 12, the cognitive ability was assessed, and the low-dose strain-treated group (G5), medium-dose strain-treated group (G6), and high-dose strain-treated group (G7) showed a concentration-dependent increase. Additionally, compared to the vehicle-treated group (G2), the high-dose strain-treated group (G7) showed a significant increase by about 2.2 times. Specifically, the donepezil-treated group (G3) showed about 74% of memory recovery, and the decursin-treated group (G4) showed about 87% of memory recovery, demonstrating that the strain of the present invention, donepezil, or decursin showed similar results in cognitive function recovery.

These results suggest that the *Lactobacillus delbrueckii* subsp. *lactis* strain of the present invention improves cognitive ability, and since this effect is similar to that of donepezil or decursin, which are AD therapeutic agents, it suggests that the strain can have a preventive, improving, or therapeutic effect on cognitive impairment or Alzheimer's disease.

### [Deposit Number]

Depository Institution: Korean Collection for Type Cultures (KCTC), a biological resource center of the Korea Research Institute of Bioscience and Biotechnology
Deposit Number: KCTC14149BP
Deposit Date: March 3, 2020

## Claims

1. A pharmaceutical composition for treating or preventing cognitive impairment or Alzheimer's disease, comprising *Lactobacillus delbrueckii* subsp. *lactis* strain deposited under deposit number KCTC14149BP as an active ingredient.

2. The pharmaceutical composition according to claim 1, wherein the strain is one or more selected from the group consisting of live cells, killed cells, and cultures, lysates, extracts, and cytoplasmic fractions thereof.

3. The pharmaceutical composition according to claim 1, wherein the cognitive impairment exhibits symptoms of decline in memory, attention, spatial perception ability, language ability, or a combination thereof.

4. The pharmaceutical composition according to claim 1, wherein the cognitive impairment is an amyloid beta protein accumulation disorder or a tau protein accumulation disorder.

5. The pharmaceutical composition according to claim 4, wherein the amyloid beta protein is a soluble or insoluble amyloid beta protein.

6. The pharmaceutical composition according to claim 1, wherein the composition shows an increase in one or more gut microbiota selected from the group consisting of *Erysipelotrichaceae, Erysipelotrichales, Turicibacter, Peptostreptococcales Tissierellales, Anaerovoracaceae* and *Eubacterium xylanophilum.*

7. The pharmaceutical composition according to claim 1, wherein the composition shows a decrease in one or more gut microbiota selected from the group consisting of *Peptoc Peptococcales* and *Peptococaceae.*

8. A food composition for alleviating or preventing cognitive impairment or Alzheimer's disease, comprising *Lactobacillus delbrueckii* subsp. *lactis* strain deposited under deposit number KCTC14149BP as an active ingredient.

9. The food composition according to claim 8, wherein the strain is one or more selected from the group consisting of live cells, killed cells, and cultures, lysates, extracts, and cytoplasmic fractions thereof.

10. The food composition according to claim 8, wherein the cognitive impairment exhibits symptoms of decline in memory, attention, spatial perception ability, language ability, or a combination thereof.
